(19) Europäisches Patentamt
European Patent Office
Office européen des brevets

(11) **EP 4 059 890 A1**

(12) **EUROPEAN PATENT APPLICATION**
published in accordance with Art. 153(4) EPC

(43) Date of publication:
**21.09.2022 Bulletin 2022/38**

(21) Application number: **20887893.4**

(22) Date of filing: **09.11.2020**

(51) International Patent Classification (IPC):
**C01F 7/00** (2022.01)   **C12M 3/00** (2006.01)
**C12N 5/071** (2010.01)   **B01J 20/08** (2006.01)

(52) Cooperative Patent Classification (CPC):
**B01J 20/08; C01F 7/00; C12M 3/00; C12N 5/06**

(86) International application number:
**PCT/JP2020/041748**

(87) International publication number:
**WO 2021/095691 (20.05.2021 Gazette 2021/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.11.2019   JP 2019205426**

(71) Applicants:
• **Nikkiso Co., Ltd.**
  **Tokyo 150-6022 (JP)**
• **Tohoku University**
  **Sendai-shi, Miyagi 980-8577 (JP)**

(72) Inventors:
• **YOSHIOKA Toshiaki**
  **Sendai-shi Miyagi 980-8577 (JP)**
• **KAMEDA Tomohito**
  **Sendai-shi Miyagi 980-8577 (JP)**
• **KITAGAWA Fumihiko**
  **Kanazawa-shi Ishikawa 920-0177 (JP)**
• **JIMBO, Yoichi**
  **Kanazawa-shi Ishikawa 920-0177 (JP)**
• **KONDO Masayuki**
  **Kanazawa-shi Ishikawa 920-0177 (JP)**

(74) Representative: **Algemeen Octrooi- en
Merkenbureau B.V.
P.O. Box 645
5600 AP Eindhoven (NL)**

(54) **LACTIC ACID ADSORPTION AGENT AND METHOD FOR REMOVING LACTIC ACID**

(57)   A lactic acid adsorption agent 4 contains at least one of: a layered double hydroxide having a plurality of metal hydroxide layers, and anions and water molecules held between the metal hydroxide layers; and a layered double oxide being an oxide of the layered double hydroxide. The metal hydroxide layers contain a divalent metal ion $M^{2+}$ and a trivalent metal ion $M^{3+}$, where the molar ratio ($M^{2+}/M^{3+}$) of the divalent metal ion $M^{2+}$ and the trivalent metal ion $M^{3+}$ in the layered double hydroxide is 1.9-3.6, and the same molar ratio in the layered double oxide is 1.8-3.6.

FIG. 1A
FIG. 1B
FIG. 1C
FIG. 1D

**Description**

TECHNICAL FIELD

The present invention relates to a lactic acid adsorbent and a method for removing lactic acid.

BACKGROUND ART

In recent years, in fields such as pharmaceutical manufacturing and regenerative medicine, artificial and efficient mass culture of cells and microorganisms has been required. Examples of cells for which mass culture is required include antibody-producing cells, such as Chinese hamster ovary cells (CHO cells), and pluripotent stem cells, such as embryonic stem cells (ES cells) and induced pluripotent stem cells (iPS cells). If such cells can be stably cultured in large quantities for a long period of time, biological substances, such as monoclonal antibodies, and differentiation induction tissues derived from pluripotent stem cells can be efficiently produced.

As a method for industrially mass culturing cells and microorganisms, suspension stirred culture with use of a culture tank, such as a spinner flask, can be considered. The suspension stirred culture, however, tends to require a large scale of equipment. Therefore, in order to reduce costs, it is effective to increase the culture density of cells and the like. It is, however, known that increasing the culture density suppresses the proliferation of cells and the like. This is because densification of cells and the like increases the concentration of waste products (metabolites) in the culture solution (liquid medium), which reduces proliferative activity of the cells and the like. As a representative waste product that affects cells and the like, lactic acid is known.

Therefore, to stably proliferate cells and the like in a high density condition, it is desirable to remove lactic acid that accumulates in the culture solution. Meanwhile, Patent Literature 1 for example discloses a cell culturing device in which a cell culturing tank and a component-controlling solution tank are connected by a feed line provided with a culture solution component-controlling membrane that allows a component to pass through depending on the concentration difference. In this cell culturing device, the waste products that have accumulated in the culture solution move to the component-controlling solution side, so that the concentration in the culture solution decreases. At the same time, the nutrients, of which the concentration has decreased during the culture process, are replenished such that nutrients in the component-controlling solution are transferred to the culture solution. The environment in the culture solution is thus maintained in a condition suitable for cell culture. As the component-controlling solution, the culture solution itself has been used.

PRIOR ART REFERENCE

PATENT LITERTURE

Patent Literature 1: WO2015/122528

SUMMARY OF INVENTION

TECHNICAL PROBLEM

In the cell culturing device disclosed in Patent Literature 1, waste products are removed from the culture solution using the principle of dialysis. Accordingly, in order to attain sufficient removal of waste products, the capacity of the component-controlling solution tank is set to 10 times or more the capacity of the cell culturing tank. There is therefore a problem that the required liquid amount becomes huge and costly. In particular, when the culture solution itself is used as the component-controlling solution, a large amount of expensive culture medium is consumed, which further increases the cost. In addition, when the waste products are removed using a dialysis technology, there is also a problem that the structure of the culturing device becomes complicated.

Therefore, a novel technology for removing lactic acid using a method other than the dialysis technology has been strongly desired. Also in such a lactic acid removal method other than the dialysis technology, high efficiency of lactic acid removal is naturally required to obtain a more favorable growth environment for cells and the like.

The present invention has been made in view of such a situation, and a purpose thereof is to provide a technology for improving the lactic acid removal efficiency.

SOLUTION TO PROBLEM

One aspect of the present invention relates to a lactic acid adsorbent. The lactic acid adsorbent includes at least one of a layered double hydroxide that contains multiple metal hydroxide layers and also contains anions and water molecules held between the metal hydroxide layers, or a layered double oxide that is an oxide of a layered double hydroxide. The metal hydroxide layers contain divalent metal ions $M^{2+}$ and trivalent metal ions $M^{3+}$, mole ratio $(M^{2+}/M^{3+})$ of the divalent metal ions $M^{2+}$ to the trivalent metal ions $M^{3+}$ in a layered double hydroxide is 1.9 to 3.6, and the mole ratio in a layered double oxide is 1.8 to 3.6.

Another aspect of the present invention relates to a method for removing lactic acid. The method for removing includes bringing the lactic acid adsorbent according to the abovementioned aspect into contact with lactic acid.

Optional combinations of the aforementioned constituting elements, and implementation of the present invention, including the constituting elements and expressions, in the form of methods, apparatuses, or systems may also be practiced as additional modes of the present invention.

ADVANTAGEOUS EFFECTS OF INVENTION

The present invention can improve the lactic acid removal efficiency.

BRIEF DESCRIPTION OF DRAWINGS

FIGS. 1A-1D are schematic diagrams used to describe methods for removing lactic acid according to an embodiment;
FIG. 2 shows a target value and an actual measured value of Mg/Al mole ratio of each of layered double hydroxides and layered double oxides;
FIG. 3 shows a lactic acid adsorption rate and a glucose adsorption rate of each of layered double hydroxides and layered double oxides in an aqueous solution containing lactic acid and glucose;
FIG. 4 shows a lactic acid adsorption rate and a glucose adsorption rate of each of the layered double hydroxides and the layered double oxides in a cell culture solution;
FIG. 5 shows lactic acid adsorption rates and glucose adsorption rates of each of a layered double hydroxide and a layered double oxide in a cell culture solution; and
FIG. 6 shows a cell proliferation rate when each of layered double hydroxides and layered double oxides is added to a culture medium.

DESCRIPTION OF EMBODIMENTS

One aspect of the present invention relates to a lactic acid adsorbent. The lactic acid adsorbent includes at least one of a layered double hydroxide that contains multiple metal hydroxide layers and also contains anions and water molecules held between the metal hydroxide layers, or a layered double oxide that is an oxide of a layered double hydroxide. The metal hydroxide layers contain divalent metal ions $M^{2+}$ and trivalent metal ions $M^{3+}$, mole ratio $(M^{2+}/M^{3+})$ of the divalent metal ions $M^{2+}$ to the trivalent metal ions $M^{3+}$ in a layered double hydroxide is 1.9 to 3.6, and the mole ratio in a layered double oxide is 1.8 to 3.6.

In the abovementioned aspect, the divalent metal ions $M^{2+}$ may be $Mg^{2+}$ or $Ca^{2+}$. Also, the lactic acid adsorbent may be brought into contact with a solution containing lactic acid to adsorb lactic acid in the solution. Also, the solution may be a culture solution for cells or microorganisms that contains glucose.

Another aspect of the present invention relates to a method for removing lactic acid. The method for removing includes bringing the lactic acid adsorbent according to any one of the abovementioned aspects into contact with lactic acid.

In the following, the present invention will be described based on a preferred embodiment with reference to the drawings. The embodiment is intended to be illustrative only and not to limit the invention, so that it should be understood that not all of the features or combinations thereof described in the embodiment are necessarily essential to the invention. Like reference characters denote like or corresponding constituting elements, members, and processes in each drawing, and repetitive description will be omitted as appropriate. Also, the scale or shape of each component shown in each drawing is defined for the sake of convenience to facilitate the explanation and is not to be regarded as limitative unless otherwise specified. Also, when the terms "first", "second", and the like are used in the present specification or claims, such terms do not imply any order or degree of importance and are used to distinguish one configuration from another. Further, in each drawing, part of members less important in describing embodiments may be omitted.

A lactic acid adsorbent according to the present embodiment includes at least one of a layered double hydroxide (LDH) or a layered double oxide (LDO). A layered double hydroxide contains multiple metal hydroxide layers, and anions and water molecules held between the metal hydroxide layers. The metal hydroxide layers contain divalent metal ions $M^{2+}$ and trivalent metal ions $M^{3+}$ as constituent metals. More specifically, a layered double hydroxide is constituted by:

host layers (metal hydroxide layers), which are octahedral layers, positively charged by $M^{3+}$ ions substituting some $M^{2+}$ ions in $M(OH)_2$ containing divalent metal; and guest layers constituted by anions, which compensate for the positive charges in the host layers, and interlayer water. Lactic acid (lactate ions) is adsorbed to a layered double hydroxide through ion exchange with the anions in the guest layers.

A layered double hydroxide is represented by the following chemical formula.

$$[M^{2+}_{1-x}M^{3+}_x(OH)_2][A^{n-}_{x/n}\cdot yH_2O]$$

In the above formula, $M^{2+}$ represents a divalent metal ion selected from a group including $Cu^{2+}$, $Mn^{2+}$, $Mg^{2+}$, $Fe^{2+}$, $Ca^{2+}$, $Ni^{2+}$, $Zn^{2+}$, $Co^{2+}$, and $Cd^{2+}$. Also, $M^{3+}$ represents a trivalent metal ion selected from a group including $Al^{3+}$, $Cr^{3+}$, $Fe^{3+}$, $Co^{3+}$, $In^{3+}$, $Mn^{3+}$, and $V^{3+}$. Also, $A^{n-}$ represents an n-valent anion selected from a group including $CO_3^{2-}$, $SO_4^{2-}$, $Cl^-$, $OH^-$, $SiO_4^{4-}$, and $NO_3^-$. Also, n is from 1 to 3, and y is from 1 to 12. Further, x is a number that satisfies mole ratio ($M^{2+}/M^{3+}$) of divalent metal ions $M^{2+}$ to trivalent metal ions $M^{3+}$ as described below.

The mole ratio of divalent metal ions $M^{2+}$ to trivalent metal ions $M^{3+}$ in a layered double hydroxide is 1.9 to 3.6. Therefore, x in the above formula is 0.217 to 0.345. By adjusting this mole ratio in the metal hydroxide layers, the charge possessed by the layered double hydroxide or the distance between the metal hydroxide layers can be changed, thereby adjusting the lactic acid adsorption capacity of the layered double hydroxide. In particular, by setting the mole ratio between 1.9 and 3.6, inclusive, the lactic acid adsorption rate of the layered double hydroxide can be increased. In addition, the layered double hydroxide can be synthesized efficiently. The mole ratio is measured using an atomic absorption photometer.

A layered double oxide is an oxide of a layered double hydroxide. A layered double oxide is obtained by calcining a layered double hydroxide at 200 degrees C to 500 degrees C, for example. When the layered double oxide is brought into contact with an aqueous lactic acid solution, the structure of the layered double hydroxide is regenerated with lactic acid adsorbed between the layers. The mole ratio of divalent metal ions $M^{2+}$ to trivalent metal ions $M^{3+}$ in a layered double oxide is 1.8 to 3.6. Also with regard to a layered double oxide, by adjusting this mole ratio in the metal hydroxide layers, the charge possessed by the layered double oxide or the distance between the metal hydroxide layers can be changed, thereby adjusting the lactic acid adsorption capacity of the layered double oxide. In particular, by setting the mole ratio between 1.8 and 3.6, inclusive, the lactic acid adsorption rate of the layered double oxide can be increased. In addition, the layered double oxide can be synthesized efficiently.

The divalent metal ions $M^{2+}$ constituting the metal hydroxide layers may preferably be $Mg^{2+}$ or $Ca^{2+}$ and more preferably be $Mg^{2+}$. Also, the trivalent metal ions $M^{3+}$ constituting the metal hydroxide layers may preferably be $Al^{3+}$. Accordingly, an Mg-Al-based LDH and an Mg-Al-based LDO may be more preferable. With these, the toxicity of the layered double hydroxide and the layered double oxide to cells and the like can be reduced. Also, multiple types of layered double hydroxides and layered double oxides containing metal hydroxide layers constituted by different metal ions and anions may be mixed and used.

By bringing a lactic acid adsorbent including at least one of the abovementioned layered double hydroxides and layered double oxides into contact with lactic acid, the lactic acid can be adsorbed to the layered double hydroxide or layered double oxide. In particular, a lactic acid adsorbent of the present embodiment can be suitably used to adsorb and remove lactic acid in a solution. In this case, by bringing the lactic acid adsorbent into contact with a solution containing lactic acid, the lactic acid in the solution can be adsorbed. Also, when the solution is a culture solution for cells or microorganisms that contains glucose, the lactic acid adsorbent can highly selectively adsorb lactic acid to be removed, compared to the glucose to be retained in the culture solution. The type of the culture solution is not particularly limited.

Also, the amount of lactic acid adsorbent added to the solution, i.e., the concentration of the lactic acid adsorbent in the solution, may preferably be more than 0.0005 g/mL, more preferably be 0.005 g/mL or more, further preferably be more than 0.005 g/mL, and yet further preferably be 0.025 g/mL or more. The amount may also preferably be less than 0.2 g/mL and more preferably be 0.1 g/mL or less. By setting the amount of the lactic acid adsorbent to be added to more than 0.0005 mg/mL, the lactic acid adsorption rate can be increased more certainly. Also, by setting the amount of the lactic acid adsorbent to be added to less than 0.2 g/mL, the glucose adsorption rate can be kept lower more certainly, so that cells and the like can be cultured more efficiently. The lactic acid adsorption rate is the ratio of the amount of adsorbed lactic acid to the total amount of lactic acid in the solution. Also, the glucose adsorption rate is the ratio of the amount of adsorbed glucose to the total amount of glucose in the solution.

The cells and microorganisms cultured using a culture solution are not particularly limited. For example, cultured cells include: pluripotent stem cells, such as human iPS cells, human ES cells, and human Muse cells, and differentiation induction cells therefrom; somatic stem cells, such as mesenchymal stem cells (MSCs) and nephron progenitor cells; tissue cells, such as human renal proximal tubule epithelial cells, human distal tubule epithelial cells, and human collecting duct epithelial cells; antibody-producing cell lines, such as human fetal renal cells (HEK293 cells); and antibody-producing cell lines derived from animals other than humans, such as Chinese hamster ovary cells (CHO cells) and insect cells (SF9 cells). Since these cells are cells for which mass culture is particularly desired, they are more preferred targets to which the lactic acid adsorbent according to the present embodiment is applied.

Method for removing lactic acid

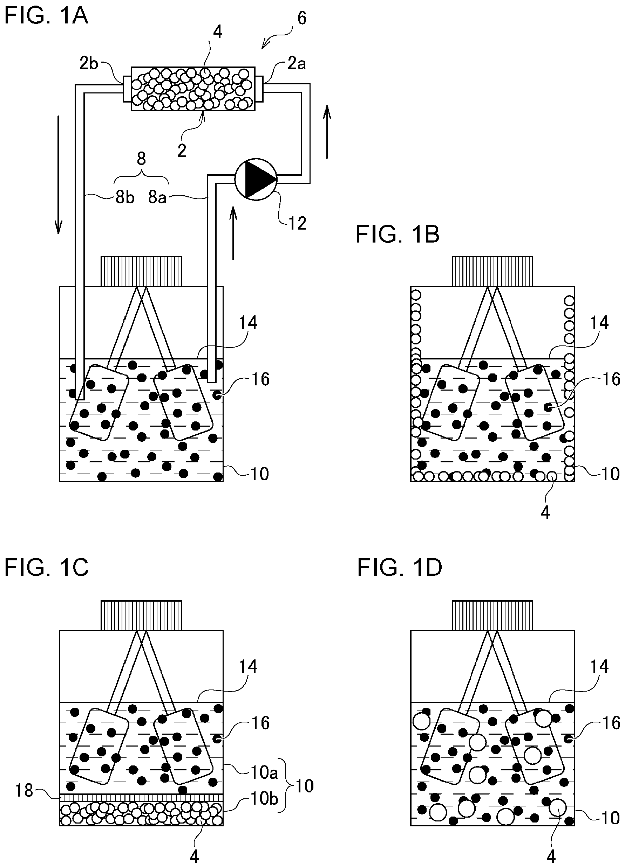

A method for removing lactic acid according to the present embodiment includes bringing the above-mentioned lactic acid adsorbent into contact with lactic acid (lactate ions). Preferably, the method involves bringing the lactic acid adsorbent into contact with a solution containing lactic acid. The method of bringing the lactic acid adsorbent into contact with lactic acid is exemplified as follows, although not particularly limited thereto. FIG. 1A to FIG. 1D are schematic diagrams used to describe methods for removing lactic acid according to the embodiment. In the following, lactic acid removal from a culture solution will be described as an example. Also removal of lactic acid from other solutions can be carried out in the same way.

In a first aspect as illustrated in FIG. 1A, an adsorption module 6 including a container 2, such as a column, filled with a lactic acid adsorbent 4 is prepared. The container 2 has an inlet 2a and an outlet 2b that each communicate with the inside and the outside of the container 2. The lactic acid adsorbent 4 may be in the form of particles, for example. The adsorption module 6 is connected, through a circulation path 8, to a culture vessel 10 such as a spinner flask. The circulation path 8 includes an outward path 8a that connects the culture vessel 10 and the inlet 2a of the container 2, and a return path 8b that connects the outlet 2b of the container 2 and the culture vessel 10. On the outward path 8a, a pump 12 is connected. The culture vessel 10 contains a culture solution 14 and cells 16. The pump 12 may alternatively be arranged on the return path 8b.

When the pump 12 is driven, the culture solution 14 is drawn from the culture vessel 10 and sent into the container 2 of the adsorption module 6 through the outward path 8a. The culture solution 14 fed into the container 2 is returned to the culture vessel 10 through the return path 8b. In the process of circulating between the culture vessel 10 and the adsorption module 6, the culture solution 14 comes into contact with the lactic acid adsorbent 4 filled in the container 2. In this process, lactic acid in the culture solution 14 is adsorbed by the lactic acid adsorbent 4. Thus, lactic acid in the culture solution 14 is removed. At an end of the outward path 8a on the side connected to the culture vessel 10, a filter, not illustrated, is provided. This prevents the cells 16 from flowing toward the adsorption module 6 side. In the process of circulating the culture solution 14 between the culture vessel 10 and the adsorption module 6, the culture solution 14 may be replenished with culture medium components, such as glucose and protein, necessary for the culture of the cells 16.

Thus, in the first aspect, lactic acid in the culture solution 14 is removed by using a culture apparatus equipped with the adsorption module 6 containing the lactic acid adsorbent 4, the culture vessel 10 containing cells or microorganisms and the culture solution 14, and the circulation path 8 connecting the adsorption module 6 and the culture vessel 10 to allow the culture solution 14 to circulate therethrough.

In a second aspect as illustrated in FIG. 1B, the lactic acid adsorbent 4 is supported on the inner wall surface of the culture vessel 10. The culture vessel 10 contains the culture solution 14 and the cells 16. The culture solution 14 therefore comes into contact with the lactic acid adsorbent 4 exposed on the inner wall surface of the culture vessel 10. Thus, the lactic acid in the culture solution 14 can be adsorbed onto the lactic acid adsorbent 4. The culture vessel 10 is exemplified by a spinner flask, petri dish, well plate, cell culture insert, and microsphere.

The method for supporting the lactic acid adsorbent 4 on the inner wall surface of the culture vessel 10 may be a method of bonding the lactic acid adsorbent 4 to the inner wall surface of the culture vessel 10 or may be, when the culture vessel 10 is made of resin, a method of molding the culture vessel 10 from a resin mixed in advance with the lactic acid adsorbent 4, for example. Thus, in the second aspect, lactic acid in the culture solution 14 is removed by using a culture apparatus equipped with the culture vessel 10, and the lactic acid adsorbent 4 supported on the inner wall surface of the culture vessel 10.

In a third aspect as illustrated in FIG. 1C, the culture vessel 10 has a structure in which the inside of the vessel is divided into an upper part 10a and a lower part 10b by a diaphragm 18 such as a porous membrane. The culture vessel 10 of this kind is exemplified by a cell culture insert. The upper part 10a contains the culture solution 14 and the cells 16, and the lower part 10b contains the culture solution 14 and the lactic acid adsorbent 4. The culture solution 14 can move back and forth between the upper part 10a and the lower part 10b through the diaphragm 18. In contrast, the cells 16 and the lactic acid adsorbent 4 cannot pass through the diaphragm 18.

In such a structure, the culture solution 14 comes into contact with the lactic acid adsorbent 4 contained in the lower part 10b. The lactic acid in the culture solution 14 can be therefore adsorbed onto the lactic acid adsorbent 4. Thus, in the third aspect, lactic acid in the culture solution 14 is removed by using a culture apparatus equipped with the culture vessel 10, the lactic acid adsorbent 4, and the diaphragm 18 that divides the inside of the culture vessel 10 into a first space containing the lactic acid adsorbent 4 and a second space containing the cells 16.

In a fourth aspect illustrated in FIG. 1D, the lactic acid adsorbent 4 in the form of particles is dispersed, precipitated, or suspended in the culture solution 14. Accordingly, the lactic acid in the culture solution 14 can be adsorbed onto the lactic acid adsorbent 4. The lactic acid adsorbent 4 preferably has predetermined size or larger, such as 10 $\mu$m or larger, to prevent phagocytosis by the cells 16. Thus, in the fourth aspect, lactic acid in the culture solution 14 is removed by using a culture apparatus equipped with the culture vessel 10, and the lactic acid adsorbent 4 added to the culture solution 14 in the culture vessel 10.

The lactic acid adsorbent is preferably coated with a resin, such as polyvinyl alcohol or alginic acid, a biological gel, such as collagen or gelatin, or the like. This can restrain fine particles that may affect cells and the like from flowing out of the lactic acid adsorbent into the culture solution. Alternatively, the lactic acid adsorbent may be formed by kneading a layered double hydroxide with a ceramic binder, resin binder, biological gel, or the like. This can also restrain the outflow of the fine particles. Examples of the ceramic binder include an alumina binder and colloidal silica. Examples of the resin binder include alginic acid, polyvinyl alcohol, and carboxymethyl cellulose. Examples of the biological gel include collagen and gelatin.

When detecting the lactic acid concentration in a solution, it is preferable to use a medium component analyzer, although the method for detection is not particularly limited. The lactic acid concentration can alternatively be detected by using a colorimetric method with a predetermined measuring reagent, an enzyme electrode method utilizing the substrate specificity of an enzyme, high performance liquid chromatography (HPLC), or the like.

As described above, a lactic acid adsorbent according to the present embodiment includes at least one of a layered double hydroxide, which contains multiple metal hydroxide layers and also contains anions and water molecules held between the metal hydroxide layers, or a layered double oxide, which is an oxide of a layered double hydroxide. The metal hydroxide layers contain divalent metal ions $M^{2+}$ and trivalent metal ions $M^{3+}$, mole ratio ($M^{2+}/M^{3+}$) of the divalent metal ions $M^{2+}$ to the trivalent metal ions $M^{3+}$ in a layered double hydroxide is 1.9 to 3.6, and the mole ratio in a layered double oxide is 1.8 to 3.6. Also, a method for removing lactic acid according to the present embodiment includes bringing the lactic acid adsorbent into contact with lactic acid.

By adjusting the mole ratio of divalent metal ions $M^{2+}$ to trivalent metal ions $M^{3+}$ in a layered double hydroxide and a layered double oxide to the above-mentioned ranges, the lactic acid adsorption rate can be increased. This can improve the lactic acid removal efficiency.

Also, according to the present embodiment, unlike the case of removing lactic acid using a conventional dialysis technology, lactic acid can be removed without using a huge amount of solution. Therefore, lactic acid can be removed at low cost. In particular, when the solution is a culture solution for cells or the like, the amount of the culture solution used can be reduced, compared to that in a conventional dialysis technology. Since culture solutions are generally expensive, further cost reduction can be achieved. Also, since lactic acid can be removed only by bringing the lactic acid adsorbent into contact with a solution containing lactic acid, the present embodiment enables simplification of the structure of a culture apparatus.

With the removal of lactic acid, cells or the like can be mass cultured densely. Also, since a decrease in pH in the culture medium caused by lactic acid can be restrained, cells can be mass cultured densely also in this respect. Further, when the cells are pluripotent stem cells, the removal of lactic acid not only enables high-density mass culture of the cells but also can maintain the undifferentiated state of the cells, i.e., the multipotency (pluripotency) of the cells. Therefore, cells suitable for production of biological substances and preparation of differentiation induction tissues can be obtained in large quantities. This can reduce the cost required for pharmaceutical manufacturing and regenerative medicine.

Also, the lactic acid adsorbent of the present embodiment can adsorb lactic acid highly selectively, compared to glucose as a useful component. This enables more efficient cell culture. Therefore, the lactic acid adsorbent of the present embodiment is particularly useful for the removal of lactic acid from a culture solution containing glucose. The lactic acid adsorbent of the present embodiment may also be used in combination with other adsorbents for other cell wastes.

Also, the divalent metal ions $M^{2+}$ constituting the metal hydroxide layers may preferably be $Mg^{2+}$ or $Ca^{2+}$. With these, the toxicity of the layered double hydroxide and the layered double oxide to cells and the like can be reduced. Therefore, a decrease in the proliferation rate of cells or microorganisms caused by the lactic acid adsorbent can be restrained. Also, the trivalent metal ions $M^{3+}$ constituting the metal hydroxide layers may preferably be $Al^{3+}$.

An embodiment of the present invention has been described in detail. The abovementioned embodiment merely describes a specific example for carrying out the present invention. The embodiment is not intended to limit the technical scope of the present invention, and various design modifications, including changes, addition, and deletion of constituting elements, may be made to the embodiment without departing from the scope of ideas of the invention defined in the claims. Such an additional embodiment with a design modification added has the effect of each of the combined embodiments and modifications. In the aforementioned embodiment, matters to which design modifications may be made are emphasized with the expression of "of the present embodiment", "in the present embodiment", or the like. However, design modifications may also be made to matters without such expression. Optional combinations of the abovementioned constituting elements may also be employed as additional aspects of the present invention.

EXAMPLES

Examples of the present invention will now be described by way of example only to suitably describe the present invention and should not be construed as limiting the scope of the invention.

Synthesis of Lactic Acid Adsorbents

Synthesis of first compound using Mg/Al mixed solution with Mg/Al mole ratio of 1.0

First, a three-neck flask containing ion-exchange water was prepared. Under nitrogen atmosphere at 30 degrees C, the ion-exchange water was stirred at 300 rpm while $Mg(NO_3)_2$-$Al(NO_3)_3$ mixed solution (Mg/Al mole ratio = 1.0) was added dropwise thereto. After one hour elapsed from the start of the drop, the reaction solution was filtered and washed with water to obtain the product. The product thus obtained was then dried at 40 degrees C under reduced pressure for 40 hours to obtain the first compound. This synthesis aims to synthesize an $NO_3$-type Mg-Al LDH with the Mg/Al mole ratio of 1.0.

Synthesis of second compound using Mg/Al mixed solution with Mg/Al mole ratio of 2.0

The second compound was synthesized using the same procedure as for the synthesis of the first compound, except that $Mg(NO_3)_2$-$Al(NO_3)_3$ mixed solution (Mg/Al mole ratio = 2.0) was used. This synthesis aims to synthesize an $NO_3$-type Mg-Al LDH with the Mg/Al mole ratio of 2.0.

Synthesis of third compound using Mg/Al mixed solution with Mg/Al mole ratio of 3.0

The third compound was synthesized using the same procedure as for the synthesis of the first compound, except that $Mg(NO_3)_2$-$Al(NO_3)_3$ mixed solution (Mg/Al mole ratio = 3.0) was used. This synthesis aims to synthesize an $NO_3$-type Mg-Al LDH with the Mg/Al mole ratio of 3.0.

Synthesis of fourth compound using Mg/Al mixed solution with Mg/Al mole ratio of 4.0

The fourth compound was synthesized using the same procedure as for the synthesis of the first compound, except that $Mg(NO_3)_2$-$Al(NO_3)_3$ mixed solution (Mg/Al mole ratio = 4.0) was used. This synthesis aims to synthesize an $NO_3$-type Mg-Al LDH with the Mg/Al mole ratio of 4.0.

Synthesis of fifth compound using Mg/Al mixed solution with Mg/Al mole ratio of 5.0

The fifth compound was synthesized using the same procedure as for the synthesis of the first compound, except that $Mg(NO_3)_2$-$Al(NO_3)_3$ mixed solution (Mg/Al mole ratio = 5.0) was used. This synthesis aims to synthesize an $NO_3$-type Mg-Al LDH with the Mg/Al mole ratio of 5.0.

Synthesis of sixth compound

The first compound was placed in an electric furnace and calcined at 500 degrees C for 2 hours to obtain the sixth compound. This synthesis aims to synthesize an Mg-Al LDO with the Mg/Al mole ratio of 1.0.

Synthesis of seventh compound

The second compound was placed in an electric furnace and calcined at 500 degrees C for 2 hours to obtain the seventh compound. This synthesis aims to synthesize an Mg-Al LDO with the Mg/Al mole ratio of 2.0.

Synthesis of eighth compound

The third compound was placed in an electric furnace and calcined at 500 degrees C for 2 hours to obtain the eighth compound. This synthesis aims to synthesize an Mg-Al LDO with the Mg/Al mole ratio of 3.0.

Synthesis of ninth compound

The fourth compound was placed in an electric furnace and calcined at 500 degrees C for 2 hours to obtain the ninth compound. This synthesis aims to synthesize an Mg-Al LDO with the Mg/Al mole ratio of 4.0.

Synthesis of tenth compound

The fifth compound was placed in an electric furnace and calcined at 500 degrees C for 2 hours to obtain the tenth compound. This synthesis aims to synthesize an Mg-Al LDO with the Mg/Al mole ratio of 5.0.

Confirmation of LDH Synthesis and LDO Synthesis

The structures of the first through tenth compounds obtained through the abovementioned synthesis procedures were confirmed by powder X-ray diffraction using an X-ray diffractometer (RINT-2200VHF, from Rigaku Corporation). With regard to the first compound synthesized targeting a layered double hydroxide with the Mg/Al mole ratio of 1.0, since multiple compounds were mixed therein, a peak derived from the layered double hydroxide alone could not be confirmed by powder X-ray diffraction (i.e., peaks derived from other compounds were also mixed). Similarly, also for the fifth compound synthesized targeting a layered double hydroxide with the Mg/Al mole ratio of 5.0, a peak derived from the layered double hydroxide alone could not be confirmed by powder X-ray diffraction. Further, also for each of the sixth compound synthesized targeting a layered double oxide with the Mg/Al mole ratio of 1.0 and the tenth compound synthesized targeting a layered double oxide with the Mg/Al mole ratio of 5.0, a peak derived from the layered double oxide alone could not be confirmed.

In other words, it was found that, in the synthesis targeting a layered double hydroxide or layered double oxide with the Mg/Al mole ratio of 1.0 and the synthesis targeting a layered double hydroxide or layered double oxide with the Mg/Al mole ratio of 5.0, the layered double hydroxide and layered double oxide could not be synthesized in a single phase. It is considered that, in the synthesis targeting a layered double hydroxide or layered double oxide with the Mg/Al mole ratio of 1.0, a layered double hydroxide or layered double oxide with the Mg/Al mole ratio around 2 was actually synthesized. Similarly, it is considered that, in the synthesis targeting a layered double hydroxide or layered double oxide with the Mg/Al mole ratio of 5.0, a layered double hydroxide or layered double oxide with the Mg/Al mole ratio around 4 was actually synthesized. It is also considered that, in such synthesis processes, impurities such as oxides of divalent metal ions increased.

Meanwhile, with regard to the second compound synthesized targeting a layered double hydroxide with the Mg/Al mole ratio of 2.0, a single peak derived from the layered double hydroxide could be confirmed by powder X-ray diffraction. Similarly, a single peak derived from a layered double hydroxide or layered double oxide could be confirmed also in each of the third compound synthesized targeting a layered double hydroxide with the Mg/Al mole ratio of 3.0, the fourth compound synthesized targeting a layered double hydroxide with the Mg/Al mole ratio of 4.0, the seventh compound synthesized targeting a layered double oxide with the Mg/Al mole ratio of 2.0, the eighth compound synthesized targeting a layered double oxide with the Mg/Al mole ratio of 3.0, and the ninth compound synthesized targeting a layered double oxide with the Mg/Al mole ratio of 4.0. In other words, it was found that, in the synthesis targeting a layered double hydroxide or layered double oxide with the Mg/Al mole ratio of 2.0, 3.0, or 4.0, the layered double hydroxide or layered double oxide could be synthesized in a single phase.

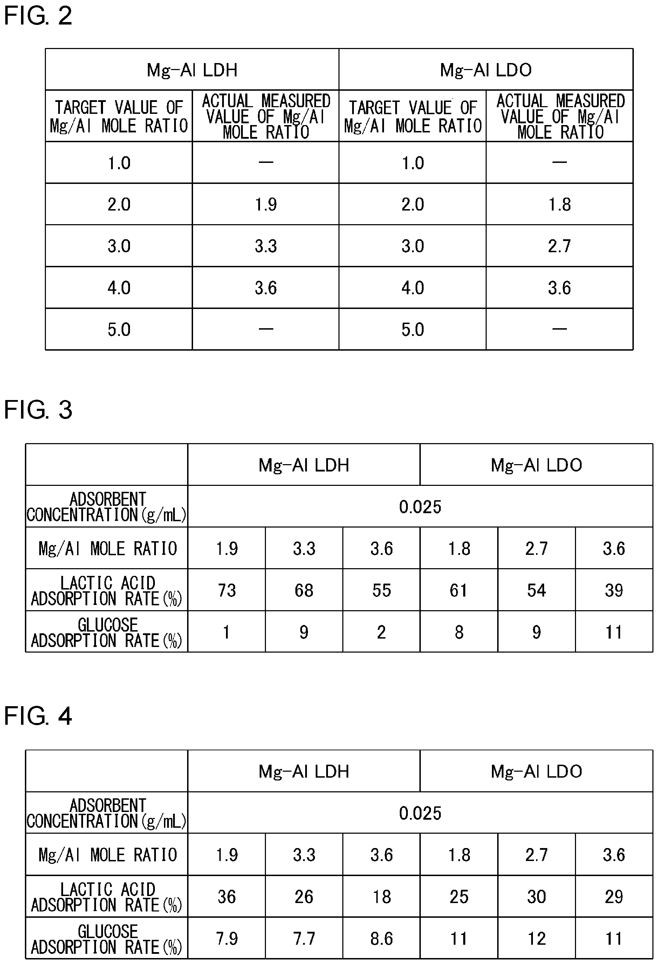

The composition of each of the synthesized layered double hydroxides and layered double oxides was confirmed using an ICP optical emission spectroscopy (iCAP6500 Duo, from Thermo Fisher Scientific Inc.). The results are shown in FIG. 2. FIG. 2 shows a target value and an actual measured value of the Mg/Al mole ratio of each of the layered double hydroxides and the layered double oxides. As shown in FIG. 2, with regard to the Mg-Al LDH synthesized targeting the Mg/Al mole ratio of 2.0, i.e., the Mg-Al LDH of which the target value of the Mg/Al mole ratio was 2.0, the actual measured value of the Mg/Al mole ratio was 1.9. Also, with regard to the Mg-Al LDH with the target value of 3.0, the actual measured value was 3.3, and, with regard to the Mg-Al LDH with the target value of 4.0, the actual measured value was 3.6. Further, with regard to the Mg-Al LDO with the target value of 2.0, the actual measured value was 1.8; with regard to the Mg-Al LDO with the target value of 3.0, the actual measured value was 2.7; and, with regard to the Mg-Al LDO with the target value of 4.0, the actual measured value was 3.6.

Performance Analyses of Adsorbents in Aqueous Solution (Water-Based Solution) Containing Lactic Acid and Glucose

Sodium lactate (Kanto Chemical Co., Inc.) and glucose (FUJIFILM Wako Pure Chemical Corporation) were added to pure water, to prepare an aqueous solution with a glucose concentration of 1000 ppm and a lactic acid concentration of 10 mM. Then, 20 mL each of the aqueous solution was dispensed into multiple 50 mL Erlenmeyer flasks. Also, 0.5 g of various lactic acid adsorbents were added respectively to the aqueous solutions in the Erlenmeyer flasks. Therefore, the concentration of each lactic acid adsorbent was 0.025 g/mL. As the lactic acid adsorbents, the aforementioned Mg/Al LDHs and Mg/Al LDOs of which the Mg/Al mole ratio could have been actually measured were used.

Each aqueous solution was then stirred at 37 degrees C and 150 rpm for 24 hours. After 24 hours, the aqueous solution and the lactic acid adsorbent were separated using a 0.1 $\mu$m filter. Thereafter, the lactic acid concentration and the glucose concentration in each aqueous solution were measured using an HPLC (JASCO Corporation). Also, the lactic acid adsorption rate and the glucose adsorption rate of each of the adsorbents were calculated based on the following formula.

```
Adsorption rate (%) = (concentration before adsorption

(mM) - concentration after adsorption (mM)) / concentration

before adsorption (mM) x 100
```

The results are shown in FIG. 3. FIG. 3 shows the lactic acid adsorption rate and the glucose adsorption rate of each of the layered double hydroxides and layered double oxides in the aqueous solution containing lactic acid and glucose. As shown in FIG. 3, it was found that each Mg-Al LDH with the Mg/Al mole ratio in the range of 1.9 to 3.6 and each Mg-Al LDO with the Mg/Al mole ratio in the range of 1.8 to 3.6 have high lactic acid adsorption capacity. In particular, among the Mg-Al LDHs, the Mg-Al LDH with the Mg/Al mole ratio of 1.9 exhibited the highest lactic acid adsorption rate. Also, among the Mg-Al LDOs, the Mg-Al LDO with the Mg/Al mole ratio of 1.8 exhibited the highest lactic acid adsorption rate. Further, it was also found that each of the Mg-Al LDHs and Mg-Al LDOs hardly adsorbs glucose in the aqueous solution.

Other compounds as described below were also synthesized and analyzed for their adsorption performance.

Synthesis of 11th compound using Cu/Al mixed solution with Cu/Al mole ratio of 3.0

A three-neck flask containing ion-exchange water was prepared. Under nitrogen atmosphere at 30 degrees C, the ion-exchange water was stirred at 300 rpm while $Cu(NO_3)_2$-$Al(NO_3)_3$ mixed solution (Cu/Al mole ratio = 3.0) was added dropwise thereto and also while the pH was adjusted to 8.0. After one hour elapsed from the start of the drop, the reaction solution was filtered and washed with water to obtain the product. The product thus obtained was then dried at 40 degrees C under reduced pressure for 40 hours to obtain the 11th compound. With regard to the 11th compound, the metal mole ratio (Cu/Al) was 3.0, the lactic acid adsorption rate was 72%, and the glucose adsorption rate was 4.2%. Thus, it was found that the 11th compound also has high lactic acid adsorption capacity.

Synthesis of 12th compound using Ni/Al mixed solution with Ni/Al mole ratio of 3.0

A three-neck flask containing ion-exchange water was prepared. Under nitrogen atmosphere at 30 degrees C, the ion-exchange water was stirred at 300 rpm while $Ni(NO_3)_2$-$Al(NO_3)_3$ mixed solution (Ni/Al mole ratio = 3.0) was added dropwise thereto and also while the pH was adjusted to 10.5. After one hour elapsed from the start of the drop, the reaction solution was filtered and washed with water to obtain the product. The product thus obtained was then dried at 40 degrees C under reduced pressure for 40 hours to obtain the 12th compound. With regard to the 12th compound, the metal mole ratio (Ni/Al) was 2.6, the lactic acid adsorption rate was 77%, and the glucose adsorption rate was 5.2%. Thus, it was found that the 12th compound also has high lactic acid adsorption capacity.

Synthesis of 13th compound using Ca/Al mixed solution with Ca/Al mole ratio of 3.0

A three-neck flask containing ion-exchange water was prepared. Under the conditions of nitrogen atmosphere, 30 degrees C, and pH 12.5, the ion-exchange water was stirred at 300 rpm while $Ca(NO_3)_2$-$Al(NO_3)_3$ mixed solution (Ca/Al mole ratio = 3.0) was added dropwise thereto. After one hour elapsed from the start of the drop, the drop was terminated, and the conditions were changed to nitrogen atmosphere, 80 degrees C, and pH 12.5. After two hours elapsed from the change of conditions, the reaction solution was filtered and washed with water to obtain the product. The product thus obtained was then dried at 40 degrees C under reduced pressure for 40 hours to obtain the 13th compound. With regard to the 13th compound, the metal mole ratio (Ca/Al) was 2.5, the lactic acid adsorption rate was 48%, and the glucose adsorption rate was 22%. Thus, it was found that the 13th compound also has high lactic acid adsorption capacity.

Performance Analyses 1 of Adsorbents in Cell Culture Solution

Sodium lactate (FUJIFILM Wako Pure Chemical Corporation) was added to a pluripotent stem cell medium (StemFit AK02N, from Ajinomoto Co., Inc.) to prepare a culture medium with a glucose concentration of 250 mg/mL and a lactic acid concentration of 10 mM. Then, 20 mL each of the culture medium was dispensed into multiple 50 mL tubes (Thermo Fisher Scientific Inc.). Also, 0.5 g of various lactic acid adsorbents were added respectively to the culture media in the tubes. Therefore, the concentration of each lactic acid adsorbent was 0.025 g/mL. As the lactic acid adsorbents, the aforementioned Mg/Al LDHs and Mg/Al LDOs of which the Mg/Al mole ratio could have been actually measured were used.

Each culture medium was then shaken at 37 degrees C and 60 times/min for 24 hours. After 24 hours, the culture medium and the lactic acid adsorbent were separated using a 0.22 $\mu$m filter. Thereafter, the lactic acid concentration and the glucose concentration in each culture medium were measured using a blood gas analyzer (ABL800 FLEX, from Radiometer Medical ApS). Also, the lactic acid adsorption rate and the glucose adsorption rate of each of the lactic acid adsorbents were calculated based on the aforementioned formula.

The results are shown in FIG. 4. FIG. 4 shows the lactic acid adsorption rate and the glucose adsorption rate of each of the layered double hydroxides and the layered double oxides in a cell culture solution. As shown in FIG. 4, although the adsorption rates were somewhat lower than those in the water-based solution, it was found that each Mg-Al LDH with the Mg/Al mole ratio in the range of 1.9 to 3.6 and each Mg-Al LDO with the Mg/Al mole ratio in the range of 1.8 to 3.6 is capable of adsorbing lactic acid also in a cell culture solution. In particular, among the Mg-Al LDHs, the Mg-Al LDH with the Mg/Al mole ratio of 1.9 exhibited the highest lactic acid adsorption rate. Also, among the Mg-Al LDOs, the Mg-Al LDO with the Mg/Al mole ratio of 2.7 exhibited the highest lactic acid adsorption rate. Further, it was also found that each of the Mg-Al LDHs and Mg-Al LDOs has a glucose adsorption rate lower than the lactic acid adsorption rate.

Performance Analyses 2 of Adsorbents in Cell Culture Solution

Sodium lactate (FUJIFILM Wako Pure Chemical Corporation) was added to a pluripotent stem cell medium (StemFit AK02N, from Ajinomoto Co., Inc.) to prepare a culture medium with a glucose concentration of 250 mg/mL and a lactic acid concentration of 10 mM. Then, 20 mL each of the culture medium was dispensed into multiple 50 mL tubes (Thermo Fisher Scientific Inc.). Also, various lactic acid adsorbents were added respectively to the culture media in the tubes. As the lactic acid adsorbents, the Mg/Al LDH of which the actual measured value of the Mg/Al mole ratio was 1.9 and the Mg/Al LDO of which the actual measured value of the Mg/Al mole ratio was 2.7 were used. The amount of each lactic acid adsorbent added (concentration) was set to 0.1 g (0.005 g/mL), 0.5 g (0.025 g/mL), 1.0 g (0.05 g/mL), and 2.0 g (0.1 g/mL).

Each culture medium was then shaken at 37 degrees C and 60 times/min for 24 hours. After 24 hours, the culture medium and the lactic acid adsorbent were separated using a 0.22 $\mu$m filter. Thereafter, the lactic acid concentration and the glucose concentration in each culture medium were measured using a blood gas analyzer (ABL800 FLEX, from Radiometer Medical ApS). Also, the lactic acid adsorption rate and the glucose adsorption rate of each of the lactic acid adsorbents were calculated based on the aforementioned formula.

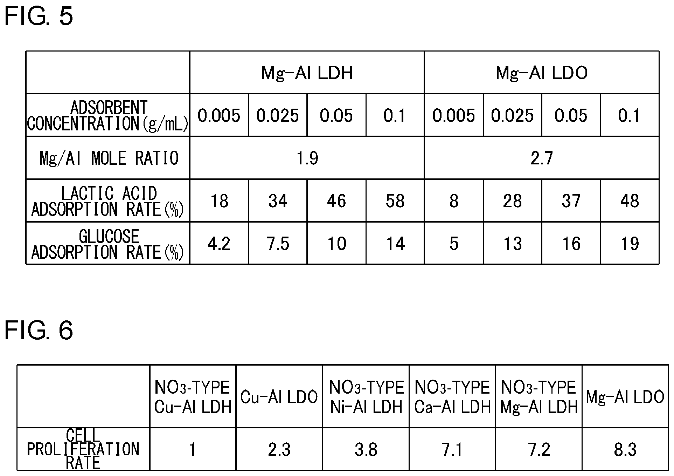

The results are shown in FIG. 5. FIG. 5 shows the lactic acid adsorption rates and the glucose adsorption rates of each of the layered double hydroxide and the layered double oxide in the cell culture solution. As shown in FIG. 5, it was found that the lactic acid adsorption rate increased as the adsorbent concentration increased. In particular, it was found that, when the adsorbent concentration was 0.025 g/mL or higher, the resulting lactic acid adsorption rate was more favorable. Although the glucose adsorption rate also increased at the same time, the glucose adsorption rate was lower than the lactic acid adsorption rate at all the adsorbent concentrations.

Toxicity Evaluation of Lactic Acid Adsorbents

Synthesis of $NO_3$-type Cu-Al LDH

A three-neck flask containing ion-exchange water was prepared. Under the conditions of nitrogen atmosphere, 30 degrees C, and pH 10.5, the ion-exchange water was stirred at 300 rpm while $Cu(NO_3)_2$-$Al(NO_3)_3$ mixed solution (Cu/Al mole ratio = 3.0) was added dropwise thereto. After one hour elapsed from the start of the drop, the reaction solution was filtered and washed with water to obtain the product. The product thus obtained was then dried at 40 degrees C under reduced pressure for 40 hours to obtain an $NO_3$-type Cu-Al LDH with $NO_3^{2-}$ as the retention ion.

Synthesis of Cu-Al LDO

The $NO_3$-type Cu-Al LDH was placed in an electric furnace and calcined at 200 degrees C for 4 hours to obtain a Cu-Al LDO.

Synthesis of NO$_3$-type Ni-Al LDH

A three-neck flask containing ion-exchange water was prepared. Under the conditions of nitrogen atmosphere, 30 degrees C, and pH 10.5, the ion-exchange water was stirred at 300 rpm while Ni(NO$_3$)$_2$-Al(NO$_3$)$_3$ mixed solution (Ni/Al mole ratio = 3.0) was added dropwise thereto. After one hour elapsed from the start of the drop, the reaction solution was filtered and washed with water to obtain the product. The product thus obtained was then dried at 40 degrees C under reduced pressure for 40 hours to obtain an NO$_3$-type Ni-Al LDH with NO$_3^{2-}$ as the retention ion.

Synthesis of NO$_3$-type Ca-Al LDH

A three-neck flask containing ion-exchange water was prepared. Under the conditions of nitrogen atmosphere, 30 degrees C, and pH 12.5, the ion-exchange water was stirred at 300 rpm while Ca(NO$_3$)$_2$-Al(NO$_3$)$_3$ mixed solution (Ca/Al mole ratio = 3.0) was added dropwise thereto. After one hour elapsed from the start of the drop, the drop was terminated, and the conditions were changed to nitrogen atmosphere, 80 degrees C, and pH 12.5. After two hours elapsed from the change of conditions, the reaction solution was filtered and washed with water to obtain the product. The product thus obtained was then dried at 40 degrees C under reduced pressure for 40 hours to obtain an NO$_3$-type Ca-Al LDH with NO$_3^-$ as the retention ion.

Thereafter, 20 mL each of a pluripotent stem cell medium (StemFit AK02N, from Ajinomoto Co., Inc.) was dispensed into multiple containers. Then, 0.5 g of various lactic acid adsorbents were added respectively to the culture media and mixed, and the resulting mixtures were left to stand at 4 degrees C for 24 hours. Therefore, the concentration of each lactic acid adsorbent was 0.025 g/mL. As the lactic acid adsorbents, the NO$_3$-type Cu-Al LDH, the Cu-Al LDO, the NO$_3$-type Ni-Al LDH, the NO$_3$-type Ca-Al LDH, the NO$_3$-type Mg/Al LDH of which the actual measured value of the Mg/Al mole ratio was 3.3, and the Mg/Al LDO of which the actual measured value of the Mg/Al mole ratio was 2.7 were used. After 24 hours, the culture medium and the lactic acid adsorbent were separated using a 0.22 μm filter.

In each culture medium, 20000 human iPS cells (201B7 cell line: Takahashi K, et al. (2007) Cell) were seeded and cultured for 120 hours. Each culture medium was replaced every 24 hours. After 120 hours elapsed, the number of cells in each culture medium was measured using the TC20 Automated Cell Counter (Bio-Rad Laboratories, Inc.). Also, the cell proliferation rate in each culture medium was calculated based on the following formula.

```
Cell proliferation rate (%) = (number of cells after

culture - number of seeded cells) / number of seeded cells x

100
```

The results showed that the cell proliferation rate in the culture medium with the NO$_3$-type Cu-Al LDH added thereto was the lowest. Based on the cell proliferation rate of the NO$_3$-type Cu-Al LDH, the ratio of the cell proliferation rate (hereinafter, simply referred to as the cell proliferation rate) in each of the culture media with the other LDHs and LDOs added respectively thereto was calculated. The results are shown in FIG. 6. FIG. 6 shows the cell proliferation rate when each of the layered double hydroxides and the layered double oxides is added to the culture medium. As shown in FIG. 6, it was found that the cell proliferation rate was higher in the order of the Mg/Al LDO, the NO$_3$-type Mg/Al LDH, the NO$_3$-type Ca-Al LDH, the NO$_3$-type Ni-Al LDH, the Cu-Al LDO, and the NO$_3$-type Cu-Al LDH.

From the above, it was found that a lactic acid adsorbent including at least one of a layered double hydroxide with mole ratio (M$^{2+}$/M$^{3+}$) of divalent metal ions M$^{2+}$ to trivalent metal ions M$^{3+}$ in the range of 1.9 to 3.6 or a layered double oxide with the mole ratio in the range of 1.8 to 3.6 exhibits excellent lactic acid adsorption capacity both in an aqueous lactic acid solution and in a culture medium. It was also found that the lactic acid adsorbent adsorbs lactic acid highly selectively, compared to glucose. The amount of glucose adsorbed by each lactic acid adsorbent in the present embodiment was at a level where the effect on the culture of cells and the like was negligible. Therefore, it was confirmed that the lactic acid adsorbents of the present embodiment are suitable for the removal of lactic acid from a culture medium containing glucose. It was also confirmed that using Mg$^{2+}$ or Ca$^{2+}$ ions, which are major metal ions included in culture mediums, as constituent metal of the metal hydroxide layers can reduce the negative effect on the proliferation of cells and the like.

INDUSTRIAL APPLICABILITY

The present invention is applicable to lactic acid adsorbents and methods for removing lactic acid.

REFERENCE SIGNS LIST

2       container
4       lactic acid adsorbent
6       adsorption module
8       circulation path
10      culture vessel
12      pump
14      culture solution
16      cell
18      diaphragm

**Claims**

1.  A lactic acid adsorbent including at least one of a layered double hydroxide that contains a plurality of metal hydroxide layers and also contains anions and water molecules held between the metal hydroxide layers, or a layered double oxide that is an oxide of the layered double hydroxide,

    the metal hydroxide layers containing divalent metal ions $M^{2+}$ and trivalent metal ions $M^{3+}$,
    mole ratio ($M^{2+}/M^{3+}$) of the divalent metal ions $M^{2+}$ to the trivalent metal ions $M^{3+}$ in the layered double hydroxide being 1.9 to 3.6, and
    the mole ratio in the layered double oxide being 1.8 to 3.6.

2.  The lactic acid adsorbent according to claim 1, wherein the divalent metal ions $M^{2+}$ are $Mg^{2+}$ or $Ca^{2+}$.

3.  The lactic acid adsorbent according to claim 1 or 2 that is brought into contact with a solution containing lactic acid to adsorb lactic acid in the solution.

4.  The lactic acid adsorbent according to claim 3, wherein the solution is a culture solution for cells or microorganisms that contains glucose.

5.  A method for removing lactic acid, the method comprising bringing the lactic acid adsorbent according to any one of claims 1 through 4 into contact with lactic acid.

FIG. 1A

FIG. 1B

FIG. 1C

FIG. 1D

## FIG. 2

| Mg-Al LDH | | Mg-Al LDO | |
|---|---|---|---|
| TARGET VALUE OF Mg/Al MOLE RATIO | ACTUAL MEASURED VALUE OF Mg/Al MOLE RATIO | TARGET VALUE OF Mg/Al MOLE RATIO | ACTUAL MEASURED VALUE OF Mg/Al MOLE RATIO |
| 1.0 | — | 1.0 | — |
| 2.0 | 1.9 | 2.0 | 1.8 |
| 3.0 | 3.3 | 3.0 | 2.7 |
| 4.0 | 3.6 | 4.0 | 3.6 |
| 5.0 | — | 5.0 | — |

## FIG. 3

| | Mg-Al LDH | | | Mg-Al LDO | | |
|---|---|---|---|---|---|---|
| ADSORBENT CONCENTRATION (g/mL) | 0.025 | | | | | |
| Mg/Al MOLE RATIO | 1.9 | 3.3 | 3.6 | 1.8 | 2.7 | 3.6 |
| LACTIC ACID ADSORPTION RATE (%) | 73 | 68 | 55 | 61 | 54 | 39 |
| GLUCOSE ADSORPTION RATE (%) | 1 | 9 | 2 | 8 | 9 | 11 |

## FIG. 4

| | Mg-Al LDH | | | Mg-Al LDO | | |
|---|---|---|---|---|---|---|
| ADSORBENT CONCENTRATION (g/mL) | 0.025 | | | | | |
| Mg/Al MOLE RATIO | 1.9 | 3.3 | 3.6 | 1.8 | 2.7 | 3.6 |
| LACTIC ACID ADSORPTION RATE (%) | 36 | 26 | 18 | 25 | 30 | 29 |
| GLUCOSE ADSORPTION RATE (%) | 7.9 | 7.7 | 8.6 | 11 | 12 | 11 |

## FIG. 5

| | Mg–Al LDH | | | | Mg–Al LDO | | | |
|---|---|---|---|---|---|---|---|---|
| ADSORBENT CONCENTRATION (g/mL) | 0.005 | 0.025 | 0.05 | 0.1 | 0.005 | 0.025 | 0.05 | 0.1 |
| Mg/Al MOLE RATIO | 1.9 | | | | 2.7 | | | |
| LACTIC ACID ADSORPTION RATE (%) | 18 | 34 | 46 | 58 | 8 | 28 | 37 | 48 |
| GLUCOSE ADSORPTION RATE (%) | 4.2 | 7.5 | 10 | 14 | 5 | 13 | 16 | 19 |

## FIG. 6

| | NO3-TYPE Cu-Al LDH | Cu–Al LDO | NO3-TYPE Ni-Al LDH | NO3-TYPE Ca-Al LDH | NO3-TYPE Mg-Al LDH | Mg-Al LDO |
|---|---|---|---|---|---|---|
| CELL PROLIFERATION RATE | 1 | 2.3 | 3.8 | 7.1 | 7.2 | 8.3 |

**INTERNATIONAL SEARCH REPORT**

| International application No. |
|---|
| PCT/JP2020/041748 |

### A. CLASSIFICATION OF SUBJECT MATTER
C01F 7/00(2006.01)i; C12M 3/00(2006.01)i; C12N 5/071(2010.01)i; B01J 20/08(2006.01)i
FI: B01J20/08 C; C12N5/071; C12M3/00 Z; C01F7/00 C
According to International Patent Classification (IPC) or to both national classification and IPC

### B. FIELDS SEARCHED
Minimum documentation searched (classification system followed by classification symbols)
C01F7/00; C12M3/00; C12N5/071; B01J20/08

Documentation searched other than minimum documentation to the extent that such documents are included in the fields searched
Published examined utility model applications of Japan        1922-1996
Published unexamined utility model applications of Japan      1971-2020
Registered utility model specifications of Japan              1996-2020
Published registered utility model applications of Japan      1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y A | JP 2016-36804 A (KANAZAWA INSTITUTE OF TECHNOLOGY) 22 March 2016 (2016-03-22) paragraphs [0023]-[0050], table 2 | 1-3, 5 4 |
| Y A | JP 58-214338 A (KYOWA CHEMICAL INDUSTRY CO., LTD.) 13 December 1983 (1983-12-13) page 3, lower right column, line 11 to page 4, upper left column, line 1, page 4, lower left column, lines 5-9 | 1-3, 5 4 |
| A | JP 2009-178682 A (NATIONAL INSTITUTE OF ADVANCED INDUSTRIAL SCIENCE AND TECHNOLOGY) 13 August 2009 (2009-08-13) entire text, all drawings | 1-5 |
| P, A | WO 2020/050068 A1 (NIKKISO CO., LTD.) 12 March 2020 (2020-03-12) entire text, all drawings | 1-5 |
| P, A | CN 111001375 A (UNIV FUJIAN TECHNOLOGY) 14 April 2020 (2020-04-14) entire text, all drawings | 1-5 |
| P, A | JP 2020-184979 A (NIKKISO CO., LTD.) 19 November 2020 (2020-11-19) entire text, all drawings | 1-5 |

☐ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 December 2020 (15.12.2020) | 28 December 2020 (28.12.2020) |

| Name and mailing address of the ISA/ Japan Patent Office 3-4-3, Kasumigaseki, Chiyoda-ku, Tokyo 100-8915, Japan | Authorized officer |
|---|---|
| | Telephone No. |

Form PCT/ISA/210 (second sheet) (January 2015)

**INTERNATIONAL SEARCH REPORT**

Information on patent family members

International application No.

PCT/JP2020/041748

| Patent Documents referred in the Report | Publication Date | Patent Family | Publication Date |
|---|---|---|---|
| JP 2016-36804 A | 22 Mar. 2016 | (Family: none) | |
| JP 58-214338 A | 13 Dec. 1983 | US 4458030 A column 3, line 46 to column 4, line 7 | |
| JP 2009-178682 A | 13 Aug. 2009 | WO 2009/096597 A1 entire text, all drawings CN 101970099 A | |
| WO 2020/050068 A1 | 12 Mar. 2020 | (Family: none) | |
| CN 111001375 A | 14 Apr. 2020 | (Family: none) | |
| JP 2020-184979 A | 19 Nov. 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015122528 A

**Non-patent literature cited in the description**

- **TAKAHASHI K et al.** *Cell,* 2007